# EUROPEAN PATENT SPECIFICATION

(11) **EP 2 632 387 B1**
(45) Date of publication and mention of the grant of the patent: **04.01.2017**
(21) Application number: 11799312.1
(22) Date of filing: 21.10.2011
(51) Int. Cl.: A61F 2/08, A61B 17/04

(54) **A DEVICE FOR FASTENING TENDONS OR LIGAMENTS, IN PARTICULAR FOR AN ANTERIOR CRUCIATE LIGAMENT OR A POSTERIOR CRUCIATE LIGAMENT**
VORRICHTUNG ZUR BEFESTIGUNG VON SEHNEN ODER BÄNDERN, INSBESONDERE FÜR EIN VORDERES KREUZBAND ODER HINTERES KREUZBAND
DISPOSITIF DE FIXATION DE TENDONS OU DE LIGAMENTS, NOTAMMENT D'UN LIGAMENT CROISÉ ANTÉRIEUR DU GENOU OU D'UN LIGAMENT CROISÉ POSTÉRIEUR DU GENOU

(30) Priority: 26.10.2010 IT PD20100323
(43) Date of publication of application: 04.09.2013
(73) Proprietor: Minozzi, Roberto, 35030 Selvazzano Dentro (PD) (IT); Lai, Lionello, 38054 Siror (TV) (IT)
(72) Inventor: Minozzi, Roberto, 35030 Selvazzano Dentro (PD) (IT); Lai, Lionello, 38054 Siror (TV) (IT)
(74) Representative: Susanetto, Carlo
(86) International application number: PCT/IB2011/054714
(87) International publication number: WO 2012/056384

(56) References cited:
- WO-A1-02/11630
- WO-A1-98/18409
- WO-A2-02/30262
- US-A- 5 584 695
- US-A1- 2002 156 476
- US-A1- 2008 033 441

## Description

### Technical field

The present invention relates to a device for fastening tendons or ligaments, in particular for an anterior cruciate ligament or a posterior cruciate ligament according to the features set out in the preamble of the main claim.

The device to which the invention relates is used preferably but not exclusively in operations which are intended to restore the functionality of the knee following tears or ruptures of the anterior cruciate ligament or posterior cruciate ligament.

### Technological background

As a result of serious tears of the ligaments, it is necessary to provide for the replacement of the damaged ligament with a ligament from a donor, optionally removed from the patient himself, or of artificial origin, which is positioned inside the joint of the knee and which is fastened to the femur and to the tibia by means of suitable fastening systems.

Typically, in order to replace the damaged cruciate ligament, the surgeon produces a tibial/femoral hole and inserts therein a prosthetic apparatus comprising a threaded bush which is intended to be screwed to the femur and an engagement element which can be associated with the threaded bush and which is provided with a slot, in which the replacement ligament is secured. After producing the tibial/femoral hole, the surgeon inserts the threaded bush therein by means of a suitable insertion instrument, screws it to the femur and, subsequently, by means of a guide rod, provides for insertion of the engagement element, to which there is secured the replacement ligament and couples it to the bush which is already in situ.

Therefore, the ligament is fastened to the tibia by means of a second screw or other suitable means.

The tension of the ligament can be subsequently adjusted by the surgeon by screwing or unscrewing the threaded capsule which, however, generates torsion of the replacement ligament which could damage it, compromising the useful life of the apparatus itself.

An example of a device for fastening ligaments of this type is set out in WO 98/18409. That device comprises a first portion which is capable of being fastened to a bone of the patient and a second portion which is stably coupled by a gripping end thereof being inserted inside the first portion. When it is stably coupled to the first portion, that is to say, when the two portions are connected so as to remain connected under normal conditions of use, with the first portion fastened to the bone and the second portion subjected to the traction of the ligament, however, the second portion cannot be rotated relative to the first portion.

In order to overcome that disadvantage, IT 1315359 has proposed an apparatus for replacement of the anterior cruciate ligament of the knee comprising a capsule which is provided with external threading which is suitable for being screwed into a portion of a tibial/femoral hole in order to fasten the apparatus itself to the femur and an appendage to which there is secured a replacement ligament, to which it is connected at the threaded capsule so as to be able to rotate freely relative thereto about the longitudinal axis of the capsule.

It is thereby possible to adjust the tension of a replacement ligament after it has been fastened to the tibia by screwing or unscrewing the capsule without subjecting the ligament to torsion.

However, both the device described in IT 1315359 and the conventional devices have the disadvantage of not allowing adequate control of the subsequent revision, or replacement of the replacement ligament.

In fact, when it becomes necessary to replace the replacement ligament, for example, owing to wear or tears thereof, it is necessary to produce another hole in the femur with a cross-section that is greater than the implant hole previously produced, or to produce another hole in order to be able to secure the new fastening apparatus to the femur.

This results in obvious discomfort for the patient, damage to the bone involved as well as difficulties of engaging the new replacement apparatus.

### Disclosure of the invention

The problem addressed by the present invention is to provide a device for fastening tendons or ligaments which is structurally and functionally configured to overcome the limits set out above with reference to the cited prior art.

Within the scope of that problem, an object of the invention is to provide a device for fastening a replacement tendon or ligament which allows easier replacement of the replacement tendon or ligament with a new tendon or ligament without damaging the implant bone and/or without any need to have to produce a new implant hole or to enlarge the hole previously used.

This problem is solved and those objects are achieved by the present invention by means of a fastening device for tendons or ligaments constructed in accordance with the appended claims.

### Brief description of the drawings

The features and advantages of the invention will be better appreciated from the detailed description of a preferred embodiment thereof which is illustrated by way of non-limiting example with reference to the appended drawings, in which:
- Figure 1 is a perspective view of a fastening device constructed according to the present invention in a first operative position,
- Figure 2 is a front cross-sectional view of the device of Figure 1,
- Figure 2a is a front cross-sectional view, drawn to an enlarged scale, of a detail of the device of Figure 1;
- Figure 3 is a front cross-sectional view of the device of Figure 1 in a second operative position;
- Figure 4 is an exploded view of the device of Figure 1;
- Figure 5 is a front view of a construction variant of the fastening device according to the present invention;
- Figure 6 is an exploded view of the device of Figure 5;
- Figure 7 is a perspective view of an insertion instrument for the device of the invention;
- Figure 8 is an exploded view of the instrument of Figure 7;
- Figure 8a is an enlarged view of a detail of Figure 8;
- Figure 9 is a view of a revision tool for the device of the invention in a first operative position;
- Figure 9a is an enlarged view of a detail of Figure 9;
- Figure 10 is an exploded view of the revision tool of Figure 9 in a second operative position;
- Figure 10a is an enlarged view of a detail of Figure 10;
- Figure 11 is a view of a construction variant of a revision tool for the device of the invention in a first operative position;
- Figure 12 is a cross-section of the revision tool of Figure 11;
- Figure 12a is an enlarged view of a detail of Figure 12.

### Preferred embodiment of the invention

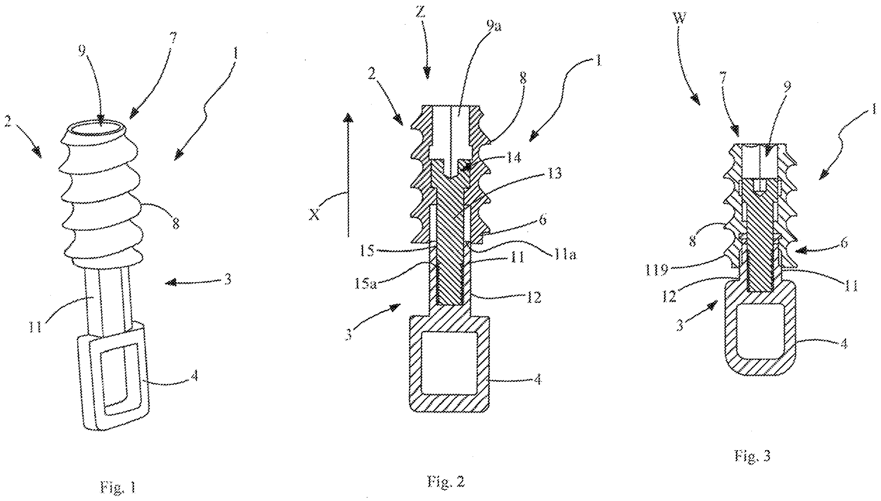

Figures 1 to 4 show a fastening device which is generally designated 1 and which is suitable for fastening a replacement tendon or ligament, in particular a ligament for replacing an anterior cruciate ligament or posterior cruciate ligament, constructed in accordance with the present invention.

The fastening device 1 comprises a first device portion 2 which is capable of being fastened to a bone, in particular a femoral bone of a person, and a second device portion 3 which is stably coupled to the first device portion 2 and which is provided with an engagement appendage and which is, for example, in the form of an eyelet 4 in order to maintain the tension of a replacement tendon or ligament, which is not shown in the Figures.

In the present description and the appended claims, the term "stably coupled" is intended to be understood to be connected so as to prevent disconnection between the two portions when they are in their normal condition of use, with the first portion fastened to a bone and the second portion fastened to the tendon or ligament.

Under normal conditions of use of the device 1, the second device portion 3 is rotatably neutral with respect to the first device portion 2 so as to allow free mutual rotation between the first device portion 2 and the second device portion 3 about a longitudinal axis X of the fastening device 1, whilst the disconnection of the two portions 2 and 3 is prevented in the longitudinal direction X as a result of the traction of the tendon or ligament, as better explained below.

The fastening device 1 further comprises fixing means 6 of the first portion 2 and/or second portion 3 which are provided in order to make the first and second device portions 2, 3 mutually integral in terms of rotation about the longitudinal axis X, as better explained below.

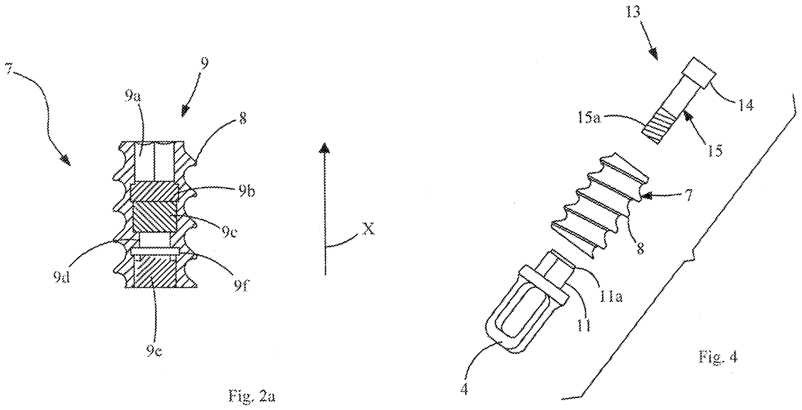

In the version shown, the first device portion 2 comprises a capsule 7 which can be better seen in Figure 2a and which is externally provided with a thread 8 which is provided to be screwed to the bone of the patient and to allow fastening of the capsule 7, and therefore of the fastening device 1, to the bone itself.

The capsule 7 is internally provided with a through-hole 9 which extends in the direction of the longitudinal axis X of the fastening device 1 and which has a plurality of sections having mutually different cross-sections.

In the version shown, the eyelet 4 of the second device portion 3 is provided with a shank 11 which extends from the eyelet 4 towards the capsule 7 and which is capable of being at least partially accommodated in the through-hole 9 of the capsule 7, as can be seen in Figure 3.

The shank 11 has a hexagonal outer cross-section and is internally provided with a threaded hole 12 which extends substantially along the longitudinal axis X coaxially with respect to the through-hole 9.

In other versions not shown, the shank 11 may have a non-hexagonal outer cross-section, for example, polygonal or elliptical. The outer cross-section of the shank 11 is selected in such a manner that, once accommodated in the through-hole 9, the free mutual rotation between the shank 11 and the capsule 7 or between the first device portion 2 and the second device portion 3 is prevented, as better explained below.

The second device portion 3 further comprises a screw 13 which extends along the longitudinal axis X and which is provided with a head 14 and a threaded shank 15.

The screw 13 is capable of being at least partially received in the through-hole 9 of the capsule 7 and the threaded hole 12 of the shank 11, so as to be operationally interposed between the eyelet 4, to which the replacement tendon or ligament is secured, and the capsule 7.

In particular in a first operative position Z shown in Figure 2, the screw 13 is positioned in such a manner that the head 14 is slidingly accommodated inside the through-hole 9 of the capsule 7 and a shank portion 15a of the threaded shank 15 protrudes from the through-hole 9 and is at least partially screwed in the threaded hole 12 of the shank 11.

In the first operative position Z, the screw 13 is free to rotate in the through-hole 9 about the longitudinal axis X with respect to the capsule 7 whilst it is screwed to, and therefore fixed for rotation with, the eyelet 4 of the second device portion 3.

The through-hole 9 of the capsule 7 has a variable cross-section along the longitudinal axis X and successively comprises a first hole section 9a which has a hexagonal cross-section and which is provided at the end of the capsule 7 intended to be directed, during use, at the opposite end with respect to the second device portion 3.

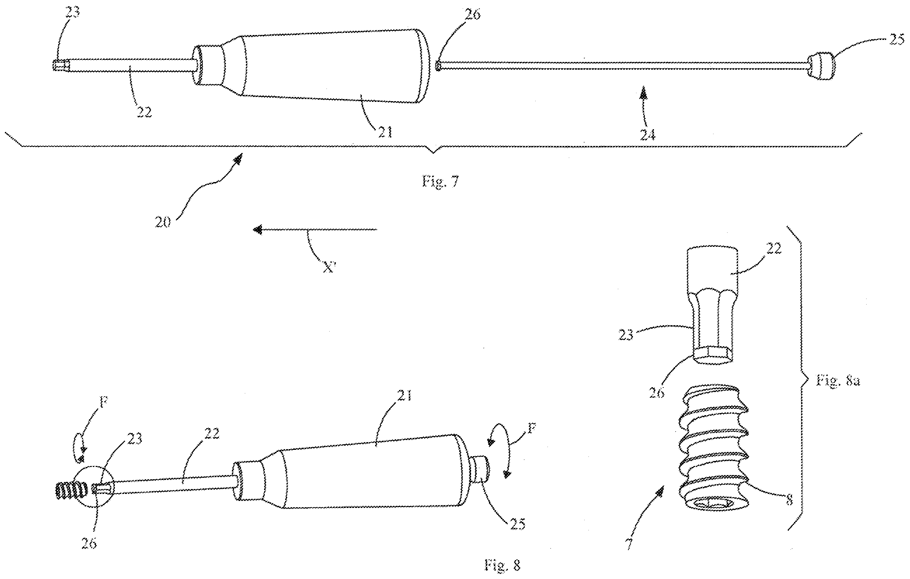

The first hole section 9a is capable of receiving an insertion instrument such as the one shown, for example, in Figures 7 and 8, in order to fasten the fastening device 1 to the bone of the patient.

In other versions which are not shown, the first hole section 9a may have a cross-section of a different shape, for example, polygonal, the first hole section 9a and the insertion instrument being mutually formed in such a manner that a portion of the insertion instrument, which will be described in greater detail below, becomes engaged therein in an anti-rotational manner, or that the capsule 7 is also caused to rotate by the above-mentioned instrument being rotated.

The through-hole 9 further comprises a second section 9b having a circular cross-section which is greater than the hexagonal cross-section of the first section 9a, at least circumscribing the hexagon defined by the cross-section of the first section 9a, so as to allow the free rotation of a shaped tip of the insertion instrument, as will be better explained below, as well as a third section 9c which is adjacent to the second section 9b and which has a circular cross-section having a diameter similar to that of the first section 9a.

The third section 9c forms a seat for accommodating the head 14 of the screw 13 that is shaped in such a manner that the screw 13 can rotate about the axis X in the above-mentioned seat without engaging with the capsule 7.

The through-hole 9 further comprises an abutment 9d, against which the head 14 of the screw 13 abuts in order to prevent it from projecting out of the through-hole 9.

The through-hole 9 further comprises a fourth section 9e which is provided at the end of the capsule 7 directed towards the second device portion 3 and which is intended to receive, in a second operative position W of the fastening device 1, at least partially the shank 11 of the eyelet 4. The fourth section 9e terminates, at the side directed towards the third section 9c, in another abutment 9f which acts as a travel stop element for the shank 11 in the through-hole 9.

The fourth section 9e has a cross-section similar to that of the shank 11, in this preferred embodiment hexagonal, in such a manner as to form, in the above-mentioned second operative position W, an anti-rotation coupling between the eyelet 4 and the capsule 7, as better explained below.

In other versions not shown, the fourth section 9e and/or the shank 11 may have a cross-section of a different shape, for example, polygonal, the fourth section 9e and/or the shank 11 being mutually formed so as to form, in the above-mentioned second operative position W, an anti-rotation coupling between the eyelet 4 and the capsule 7.

The previously described portions of the device 1 are mutually assembled in a first operative position Z shown in Figure 2, in which the screw 13 is partially inserted in the through-hole 9 and is free to rotate therein whilst the shank portion 15a which protrudes from the through-hole 9 is screwed in the threaded hole 12 of the eyelet 4 and the shank 11 of the eyelet 4 is positioned outside the through-hole 9 of the capsule 7.

In the first operative position Z, as mentioned, the first device portion 2 and the second device portion 3 are free to rotate mutually about the longitudinal axis X and are capable of mutual axial translation, even though limited, along the above-mentioned longitudinal axis X, but with the condition of stable coupling being maintained, owing to the travel stop function of the abutments 9d and 9f.

In a version which is not shown, in the first operative position Z, the first device portion 2 and the second device portion 3 are capable of mutual translation, even though limited, along an axis which is transverse with respect to the longitudinal axis X, for example, perpendicular to the longitudinal axis X.

The device 1 can further be moved from the first operative position Z to a second operative position W shown in Figure 3, and vice versa.

The eyelet 4 of the second device portion 3 can be moved towards the capsule 7 so as to progressively introduce the shank 11 into the through-hole 9, in particular in the fourth section 9e thereof, until the free end 11a of the shank 11 is brought into abutment against the additional abutment 9f of the through-hole 9 which, as mentioned, acts as a travel stop element for the shank 11.

That movement brings about consequent translation of the screw 13 whose head 14 is urged away from the abutment 9d.

As mentioned above, the outer cross-section of the shank 11 and the fourth section 9e are formed in such a manner that, in the second operative position W, the first device portion 2 and the second device portion 3 are fixed for rotation with each other about the longitudinal axis X. Therefore, the eyelet 4 being rotated also causes rotation of the capsule 7, and vice versa.

In other words, in the second operative position W, the shank 11 and the fourth section 9e act as fixing means and counter- fixing means 6 for the first device portion 2 and the second device portion 3 which co-operate in order to make them fixed for rotation with each other about the longitudinal axis X. In order to assemble the fastening device 1 of the invention, the screw 13 is inserted into the through-hole 9 at the side of the first section 9a, causing it to slide until the head 14 thereof moves into abutment with the abutment 9b.

In that position, as mentioned, a portion 15a of the threaded shank 15 of the screw 13 projects out of the through-hole 9 and is screwed into the threaded hole 12 of the shank 11 of the eyelet 4. In that manner, the first device portion 2 and the second device portion 3 are connected to each other. Therefore, the replacement tendon or ligament is secured to the eyelet 4 and the fastening device 1 assembled in that manner is inserted into the femoral/tibial hole previously formed, as will be better explained below.

In the preferred embodiment described here, the fastening device is composed of titanium, but other suitable materials may be used.

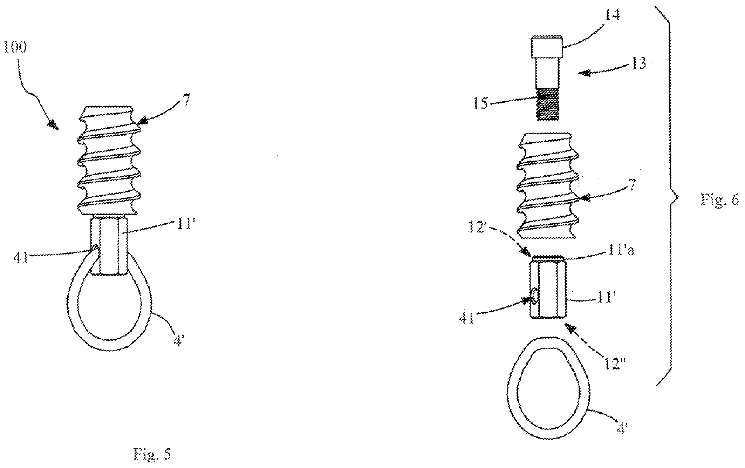

Figures 5 and 6 show an alternative variant 100 of the fastening device of the invention, in which details corresponding to the variant of Figures 1 and 4 are indicated using the same reference numerals and are not described in detail.

In the above-mentioned construction variant 100, the eyelet 4' is constituted by an annular element of practically ovoid form which is inserted into an opening 41 which is formed in the shank 11' at the side opposite the capsule 7. The shank 11' has, in the version shown, a hexagonal outer cross-section but, in other embodiments, the shank 11' may be constructed with outer cross-sections having a different shape, for example, polygonal, which is formed so as to engage in an anti-rotational manner in the fourth hole section 9e and with an end for engagement with a suitable revision tool, as will be described in greater detail below.

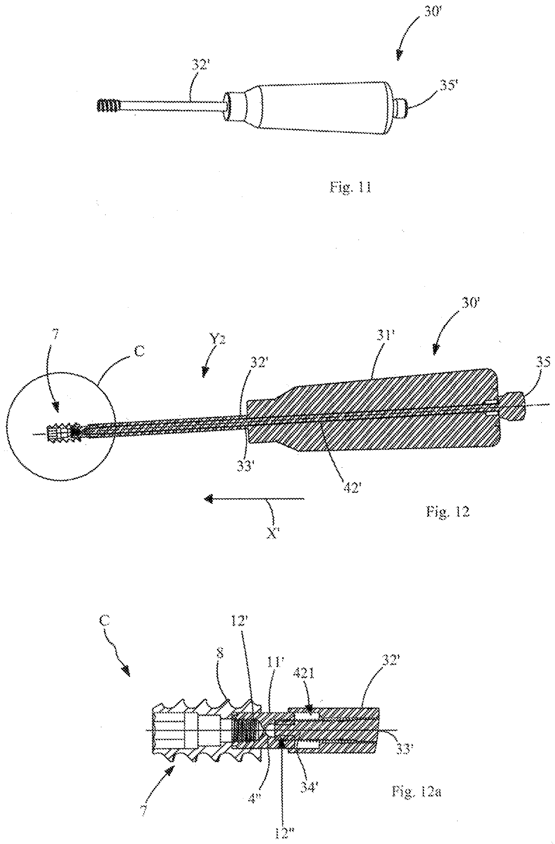

The shank 11' is internally provided, as can be seen more clearly in Figure 12a, with a threaded hole 12' which is provided on the shank portion 11' which is intended to be directed towards the first device portion 2 and with another threaded hole 12" which is provided at the opposite end of the shank 11' and which is intended to receive a portion of a revision tool, as better explained below.

In that variant, the eyelet 4' is preferably constructed from polymer material which is flexible but mechanically very strong, such as, for example, drawn polyolefin fibres or polyaramide, whilst the other portions of the device 100 may be constructed, for example, from titanium, as in the previously described version.

Figures 7 and 8 show an insertion instrument 20 which is provided to interact with the first portion 2 of the fastening device 1 in order to insert it into the desired bone seat.

The insertion instrument 20 comprises a handle 21 which can be grasped by an operator in order to manipulate the instrument 20 and a rod-like element 22 which is fastened to the handle 21 and which terminates at the opposite end with respect thereto in a tip 23, which can better be seen in Figure 8a, having a hexagonal outer cross-section which is formed so as to engage with the first section 9a of the through-hole 9 in order to cause the capsule 7 to rotate by the insertion instrument 20 itself being rotated.

In other versions not shown, as mentioned, the tip 23 may have a non-hexagonal outer cross-section, for example, polygonal or elliptical, which is formed so as to engage with the first section 9a of the through-hole 9 in order to rotate the capsule 7.

The handle 21 and the rod-like element 22 are internally hollow so that a bar-like element 24 which can be seen more clearly in Figure 7 can be accommodated therein. The bar-like element 24 is provided at opposite ends with a grip 25 which is positioned externally in abutment with the handle 21 at the opposite end with respect to the rod-like element 22, and a shaped tip 26 which is positioned externally in abutment with the tip 23.

The shaped tip 26 has an outer surface which is substantially coincident with the outer surface of the tip 23.

The bar-like element 24 can rotate about the longitudinal axis X' of the instrument 20 inside the handle 21 and the rod-like element 22 as a result of a movement of the grip 25. The grip 25 is intended to be rotated by the user in both directions of the arrow F in order consequently to rotate the shaped tip 26 so as to alternately bring it into alignment or out of alignment (Figure 8a) with the tip 23, as will be better explained below.

In the alignment position, the cross-section volume of the tip 23 and the shaped tip 26 are substantially mutually coincident whilst, in the non-aligned position, some portions of the shaped tip 26 project out of the volume of the cross-section of the tip 23.

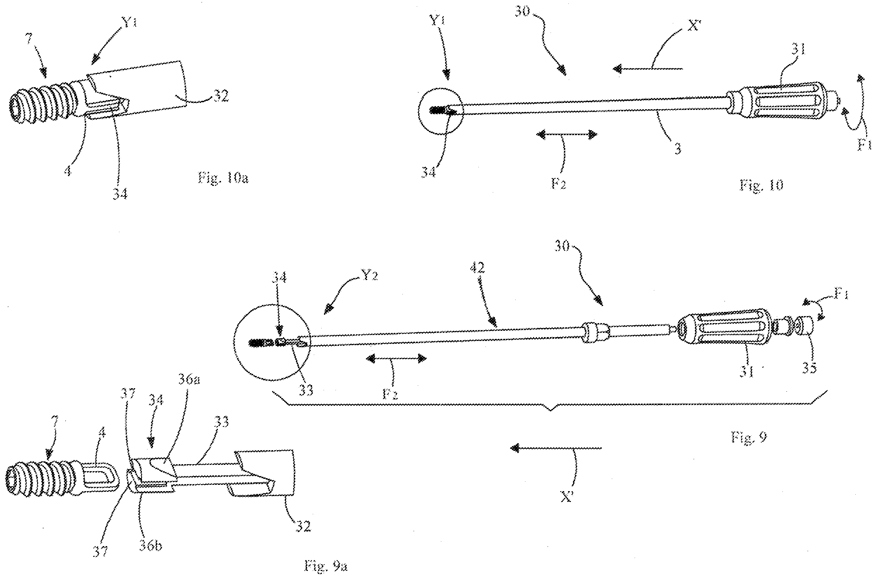

Figures 9 and 10 show a revision tool 30 which is provided to interact with the second portion 3 of the fastening device 1, in particular in the variant shown in Figures 1 - 4, if it becomes necessary, for example, for a subsequent tear of the replacement tendon or ligament, to remove the fastening device 1 in order to replace the damaged tendon or ligament.

The revision tool 30 comprises a handle portion 31 which can be grasped by an operator in order to manipulate the tool 30 and a rod-shaped body 32 which is fixed to the handle portion 31 which is provided with an internal cavity 42, in which there is slidingly inserted a rod 33 which is provided with a gripping end 34, which can be seen more clearly in Figures 9a and 10a.

The gripping end 34 comprises a pair of gripping arms 36a and 36b which are articulated to the bar 33 and which can be moved away from each other (Figure 9a) so that an engagement cavity remains defined between the two gripping arms 36a and 36b in order to insert, for example, the eyelet 4 of the second portion 3 of the fastening device 1, and moved together (Figure 10a) in order to grip the eyelet 4 between the two gripping arms 36a and 36b. Each of the facing surfaces of the two gripping arms 36a and 36b is provided with a respective protrusion 37 which is intended to move into abutment, in the moved-together position of the two gripping arms 36a, 36b, with the protrusion 37 of the opposite gripping arm so as to improve the gripping of the eyelet 4.

The revision tool 30 further comprises a washer 35 which can be positioned externally in abutment against the handle portion 31 which is at the opposite end with respect to the rod-shaped body 32 and which is intended to be rotated, by the user, in both directions of the arrow F1 in Figure 9 and Figure 10 in order to vary the screwing of the bar 33 in the washer 35 and, therefore, in order to generate relative movement between the bar 33 and the rod-shaped body 32 substantially along the longitudinal axis X' of the tool 30, as indicated by the arrow F2.

By the washer 35 being rotated, the revision tool 30 moves between a retracted position Y1, which is shown in Figure 10 and Figure 10a and in which the bar 33 is positioned almost completely inside the rod-shaped body 32, and an extended position Y2, which is shown in Figure 9 and Figure 9a and in which the gripping arms 36a and 36b of the gripping end 34 are positioned externally with respect to the rod-shaped body 32, and vice versa.

In the extended position Y2, the two gripping arms 36a and 36b are moved apart from each other so that, for example, a portion of the eyelet 4 can be freely inserted in the engagement cavity which is defined therebetween.

In the retracted position Y1, the two gripping arms 36a and 36b are moved together, the two protrusions 37 are in abutment against each other so as to grip the eyelet 4: by the washer 35 being rotated so as to move the rod-shaped body 32 and the bar 33 relative to each other in order to bring the revision tool 30 into a retracted position Y1, the walls of the rod-shaped body 32 apply a pressure to the two gripping arms 36a and 36b which tends to progressively move together the two gripping arms 36a and 36b until they have been moved into the above-mentioned position moved together. Therefore, the eyelet 4 is progressively gripped between the two gripping arms 36a and 36b and the arms, when they are in the moved-together position, grip the eyelet 4 which can therefore be moved in translation and/or rotation with respect to the axis X or, in general, along the axis along which a given mutual translation is permitted between the first device portion 2 and the second device portion 3 by means of the revision tool 30.

Figures 11 and 12 show a construction variant 30' of the revision tool which is suitable for being used with the variant of the second device portion 3 shown in Figures 5 and 6. The details corresponding to the variant of Figures 9 and 10 are indicated with the same reference numerals and are not described in detail.

The cavity 42' which is defined by the rod-shaped body 32' comprises, at the opposite end with respect to the handle portion 31', a terminal portion 421 which has an enlarged cross-section and which is formed so as to receive with anti-rotation coupling the shank 11' of the second device portion 3, as will be explained more clearly below, in order to engage the revision tool 30' and the second device portion 3 with each other.

The sliding bar 33' is provided with a threaded end 34 which can be seen more clearly in Figure 12a and which is intended to be screwed, with the washer 35' being acted upon, into the additional threaded hole 12" of the shank 11' in order to improve the mutual engagement between the revision tool 30' and the second device portion 3. In order to replace a ligament of a patient using the fastening device of the invention, a tibial/femoral hole is first formed, the insertion instrument 20 is inserted at the femur side, with the tip 23 and the shaped tip 26 in mutual alignment, through the tibial/femoral hole until engagement with the fastening device 1 which is provided at the tibia side. The tip 23 of the insertion instrument 20 is inserted into the through-hole 9 of the capsule 7 until the shaped tip 26 is moved into abutment against the head 14 of the screw 13. In that manner, the shaped tip 26 is accommodated in correspondence with the second section 9b and the tip 23 is accommodated in the first section 9a of the through-hole 9.

Subsequently, the handle 25 is rotated through a suitable angle in order to misalign the shaped tip 26 with respect to the tip 23 and, therefore, with respect to the first section 9a so that some portions of the shaped tip 26 protrude from the volume of the cross-section of the tip 23, preventing the shaped tip 26 from being able to be introduced into the first section 9a.

In that manner, the shaped tip 26 is locked inside the second section 9b, preventing the possibility that the insertion instrument 20 can be withdrawn from the through-hole 9.

Therefore, the device 1 assembled as described above is dragged by means of the insertion instrument 20 through the above-mentioned tibial/femoral hole as far as the height of the femur, where, by acting on the handle 21 in order to rotate the instrument 20, the capsule 7 is screwed to the spongy portion of the femur of the patient.

The ligament is locked in the tibial/femoral hole by means of suitable fastening means, such as, for example, a screw which extends substantially along the longitudinal axis of the hole and which locks the ligament by interference.

The adjustment of the tension of the ligament is brought about by varying the screwing of the capsule 7 to the bone, preventing occurrences of torsion of the replacement ligament by means of the freely rotational connection between the first device portion 2 and the second device portion 3.

If it becomes necessary to replace the replacement tendon or ligament to remove the fastening device 1, the surgeon makes provision for reopening the hole at the tibial side in order to access the fastening device 1.

If use is made of a fastening device having the second device portion 3, as in Figures 1 to 4, the revision tool 30 shown in Figures 9 and 10 is used.

The revision tool 30 is inserted into the above-mentioned hole with the gripping arms 36a and 36b in the extended position Y2 and is moved until the gripping arms 36a and 36b are moved into correspondence with the eyelet 4. In that position, the washer 35 is screwed by moving the bar 33 and the rod-shaped body 32 relative to each other in order to progressively move the tool into the retracted position Y1 and thereby progressively gripping the eyelet 4 between the gripping arms 36a and 36b.

After gripping the eyelet 4 between the two gripping arms 36a and 36b, the surgeon makes provision, by means of the revision tool 30, for urging the second device portion 3 towards the first device portion 2 along the longitudinal axis X of the fastening device 1 by inserting the shank 11 in the fourth hole section 9e or in the portion of the through-hole 9 defining the anti-rotation accommodating cavity for the shank 11 of the second device portion 3, so as to cause the first device portion 2 and the second device portion 3 to be fixed for rotation with each other.

Consequently, by the revision tool 30 being rotated, the capsule 7 is unscrewed from the femur in order to be able to withdraw the fastening device 1 from the tibial/femoral hole.

In order to replace the replacement tendon or ligament in the variant of the second device portion 3 shown in Figures 5 and 6, there is provision, after reopening the hole at the tibial side, for separating the eyelet 4' from the device 100 so that the shank 11' becomes readily accessible. Subsequently, the revision tool 30' is inserted into the tibial/femoral hole in such a manner that the shank 11' is gradually inserted into the terminal portion 421 of the tool 30' itself.

In that manner, the fastening device 100 is stably engaged in the revision tool 30'.

Subsequently, in order to further improve the mutual engagement between the revision tool 30'and the fastening device 100, there is provision for screwing, by acting on the washer 35', the threaded end 34' into the additional threaded hole 12" of the shank 11'.

Subsequently, steps are carried out as previously described in order to remove the fastening device 100.

In that manner, the replacement of the fastening device and/or the tendon or ligament associated therewith is carried out without substantial damage to the bone of the patient.

Therefore, the present invention solves the problem set out above with reference to the cited prior art, at the same time affording a large number of other advantages, including easy replacement of the replaced tendon or ligament with a new tendon or ligament without damaging the implant bone and/or without any need for having to produce a new implant hole and for enlarging the previously used hole.

The device of the invention prevents a large number of inconveniences for the patient, damage to the bone involved by the implant and, furthermore, optimum engagement of the new replacement apparatus is also ensured.

## Claims

1. A fastening device for a tendon or ligament, comprising:
- a first device portion (2) suitable for being fastened to a bone of a person,
- a second device portion (3) provided with an engagement appendage (4; 4') for maintaining the tension of the tendon or ligament to be fastened, and stably coupled to the first device portion (2) in order to prevent the disengagement of the tendon or ligament from the bone,
- the second portion (3) being coupled to the first portion (2) in a freely rotatable manner in order to allow free relative rotation between the first (2) and the second (3) portion about a longitudinal axis (X) of the device (1), **characterized in that** the second portion (3) is translatable in a limited manner with respect to the first portion (2) between a first operative position (Z) in which the second portion (3) is freely rotatable with respect to the first portion (2) and a second operative position (W) in which the second portion (3) is fixed for rotation with the first portion (2) and **in that** it comprises fixing means, comprising an external surface of a shank (11, 11') of the second portion (3), and corresponding counter-fixing means, comprising an accommodating cavity (9e) for the shank (11; 11'), defined in the first portion (2), the shank (11; 11') being at least partially inserted, in the second position (W), in anti-rotational engagement in the accommodating cavity (9e), in order to fix the first (2) and the second (3) portion to each other for rotation about the longitudinal axis (X).

2. A fastening device according to claim 1, wherein the second portion (3) is translatable in a limited manner along the longitudinal axis (X) in order to be moved between the first operative position (Z) and the second operative position (W).

3. A fastening device according to claim 1, wherein the first portion (2) comprises a capsule (7) extending along the longitudinal axis (X) and provided with an external thread (8) for screwing the capsule (7) to a bone of a patient, and with a through-hole (9) having a variable cross-section along the longitudinal axis (X), the accommodating cavity being defined in a section (9e) of the through-hole (9).

4. A fastening device according to the preceding claim, wherein the shank (11; 11') extends from the engagement appendage (4; 4') and is provided with a threaded hole (12), the second device portion (3) also comprising a screw (13) suitable for being inserted in a freely rotatable manner in the through-hole (9) and provided with a shank (15) suitable for being at least partially screwed into the threaded hole (12) in such a manner as to couple the second portion (3) and the first portion (2) to each other.

5. A fastening device according to any one of claims 3 or 4, wherein the shank (11; 11') and the section (9e) of the through-hole (9) have similar non-circular cross-sections so that, when the shank (11; 11') is accommodated in the section (9e), the first (2) and the second (3) device portion are fixed to each other for rotation about the longitudinal axis (X).

6. A fastening device according to any one of claims 3 to 5, wherein the through-hole (9) comprises a first section (9a) which is provided at the opposite end with respect to the second portion (3) and which is intended to receive a tip (23) of an insertion instrument (20), the first section (9a) and the tip (23) being configured in such a manner as to engage with each other in anti-rotation coupling.

7. A fastening device according to the preceding claim, wherein the through-hole (9) also comprises a second section (9b) adjacent to the first section (9a) and having a circular cross-section at least circumscribing the cross-section of the first section (9a), in such a manner as to receive a shaped tip (26) of the insertion instrument (20) and to lock the shaped tip (26) in translation with respect to the device (1).

8. A fastening device according to any one of the preceding claims, wherein the engagement appendage is in the form of an eyelet (4, 4') fastened to the shank (11; 11').

9. A fastening device according to any one of claims 1 to 7, wherein the engagement appendage is in the form of a ring (4') inserted in a hole (41) provided in the shank (11').

10. An insertion instrument for inserting a fastening device (1; 100) for a tendon or ligament according to any one of the preceding claims, comprising an insertion end (23, 26) arranged to be inserted into a through-hole (9) of a first portion (2) of the device (1; 100), the insertion end comprising a tip (23) having a non-circular cross-section configured in such a manner as to engage a first section (9a) of the hole (9), and a shaped tip (26), coaxial with the tip (23) and having a non-circular cross-section corresponding to the cross-section of the tip (23), which is intended to be accommodated rotatably in a second section (9b) of the hole (9), the shaped tip (26) being rotatable with respect to the tip (23) between a position of alignment in which the tip (23) and the shaped tip (26) are aligned in order to be inserted/extracted from the hole (9) and a position of non-alignment in which the tip (23) and the shaped tip (26) are not aligned, portions of the shaped tip (26) projecting with respect to the cross-section of the tip (23) and the shaped tip (26) being locked in the second section (9b).

11. A revision tool (30) for the fastening device (1) for a tendon or ligament according to any one of claims 1 to 9, comprising a body (32) which is rod-shaped and internally hollow in order to accommodate in a slidable manner a rod (33) provided with a gripping end (34) which is intended to interact with the second device portion (3), the gripping end (34) comprising a pair of gripping arms (36a, 36b) capable of being moved away from/towards each other, the rod-shaped body (32) and the rod (33) being movable relative to each other along a longitudinal axis (X') of the tool between an extracted position (Y2) in which the gripping end (34) is located outside the rod-shaped body (32) and the gripping arms (36a, 36b) are moved away from each other in such a manner that there is defined therebetween a seat for accommodating one end (4) of the second device portion (3), and a retracted position (Y1) in which the rod (33) is positioned almost completely inside the rod-shaped body (32) and the gripping arms (36a, 36b) have been moved towards each other in order to clamp the end (4) of the second (3) device portion.

12. A revision tool (30') for the fastening device (100) for a tendon or ligament according to any one of claims 1 to 7, or 9, comprising a handle portion (31') which is intended to be grasped by the user in order to manipulate the tool, a rod-shaped body (32') defining an internal cavity (42') for receiving in a slidable manner a rod (33') provided with a threaded end (34') which is intended to interact with the second device portion (3), the cavity (42') comprising, at the opposite end with respect to the handle portion (31'), a terminal portion (421) of enlarged cross-section configured in such a manner as to receive with anti-rotation coupling the shank (11') of the second device portion (3), the threaded end (34') being intended to be screwed into a threaded hole (12") of the shank (11').

## Patentansprüche

1. Befestigungsvorrichtung für eine Sehne oder ein Band, umfassend:
- einen ersten Vorrichtungsbereich (2), der zum Befestigen an einem Knochen einer Person geeignet ist,
- einen zweiten Vorrichtungsbereich (3), der mit einem Eingriffsansatz (4; 4') zum Aufrechterhalten der Spannung der zu befestigenden Sehne oder des zu befestigenden Bands vorgesehen ist, und mit dem ersten Vorrichtungsbereich (2) stabil verbunden ist, um das Lösen der Sehne oder des Bands vom Knochen zu verhindern,
- wobei der zweite Bereich (3) mit dem ersten Bereich (2) frei drehbar verbunden ist, um eine freie Relativdrehung zwischen dem ersten (2) und dem zweiten (3) Bereich um eine Längsachse (X) der Vorrichtung (1) zu ermöglichen,
**dadurch gekennzeichnet, dass** der zweite Bereich (3) in Bezug auf den ersten Bereich (2) zwischen einer ersten wirksamen Position (Z), an welcher der zweite Bereich (3) in Bezug auf den ersten Bereich (2) frei drehbar ist, und einer zweiten wirksamen Position (W) begrenzt verschiebbar ist, an welcher der zweite Bereich (3) zur Drehung mit dem ersten Bereich (2) fixiert ist, und dadurch, dass dieser eine Befestigungseinrichtung, die eine Außenfläche eines Schafts (11, 11') des zweiten Bereichs (3) aufweist, und eine entsprechende Gegenbefestigungseinrichtung umfasst, die einen Aufnahmehohlraum (9e) für den Schaft (11; 11') aufweist, der im ersten Bereich (2) definiert ist, wobei der Schaft (11; 11') zumindest teilweise (W) in verdrehsicherem Eingriff in die zweite Position im Aufnahmehohlraum (9e) eingeführt ist, um den ersten (2) und den zweiten (3) Bereich zur Drehung um die Längsachse (X) aneinander zu fixieren.

2. Befestigungsvorrichtung nach Anspruch 1, wobei der zweite Bereich (3) entlang der Längsachse (X) begrenzt verschiebbar ist, um zwischen der ersten wirksamen Position (Z) und der zweiten wirksamen Position (W) bewegt zu werden.

3. Befestigungsvorrichtung nach Anspruch 1, wobei der erste Bereich (2) eine Kapsel (7) aufweist, die sich entlang der Längsachse (X) erstreckt und mit einem Außengewinde zum Einschrauben der Kapsel (7) in einem Knochen eines Patienten und mit einer Durchgangsöffnung (9) versehen ist, die einen variablen Querschnitt entlang der Längsachse (X) aufweist, wobei der Aufnahmehohlraum in einem Abschnitt (9e) der Durchgangsöffnung (9) definiert ist.

4. Befestigungsvorrichtung nach dem vorhergehenden Anspruch, wobei sich der Schaft (11; 11') vom Eingriffsansatz (4; 4') erstreckt und mit einer Gewindebohrung (12) versehen ist, wobei der zweite Vorrichtungsbereich (3) ferner eine Schraube (13) aufweist, die zum frei drehbaren Einführen in die Durchgangsöffnung (9) geeignet ist und mit einem Schaft (15) versehen ist, der zum zumindest teilweisen Einschrauben in die Gewindebohrung (12) derart geeignet ist, dass dieser den zweiten Bereich (3) und den ersten Bereich (2) miteinander zu verbindet.

5. Befestigungsvorrichtung nach einem der Ansprüche 3 oder 4, wobei der Schaft (11; 11') und der Abschnitt (9e) der Durchgangsöffnung (9) ähnliche nicht-kreisförmige Querschnitte aufweisen, sodass, wenn der Schaft (11; 11') im Abschnitt (9e) aufgenommen ist, der erste (2) und der zweite (3) Vorrichtungsbereich zur Drehung um die Längsachse (X) aneinander fixiert sind.

6. Befestigungsvorrichtung nach einem der Ansprüche 3 bis 5, wobei die Durchgangsöffnung (9) einen ersten Abschnitt (9a) aufweist, der am gegenüberliegenden Ende in Bezug auf den zweiten Bereich (3) vorgesehen ist und der eine Spitze (23) eines Einführinstruments (20) aufnehmen soll, wobei der erste Abschnitt (9a) und die Spitze (23) derart konfiguriert sind, dass diese miteinander in einer Verdrehsicherungverbindung im Eingriff stehen.

7. Befestigungsvorrichtung nach dem vorhergehenden Anspruch, wobei die Durchgangsöffnung (9) ferner einen zweiten Abschnitt (9b) benachbart zum ersten Abschnitt (9a) und mit einem kreisförmigen Querschnitt aufweist, der zumindest den Querschnitt des ersten Abschnitts (9a) derart eingrenzt, dass dieser eine geformte Spitze (26) des Einführinstruments (20) aufnimmt und die geformte Spitze (26) bei einer Translation in Bezug auf die Vorrichtung verriegelt.

8. Befestigungsvorrichtung nach einem der vorhergehenden Ansprüche, wobei der Eingriffsansatz in Form einer am Schaft (11; 11') befestigten Öse (4, 4') vorliegt.

9. Befestigungsvorrichtung nach einem der Ansprüche 1 bis 7, wobei der Eingriffsansatz in Form eines Rings (4') vorliegt, der in eine im Schaft (11') vorgesehene Öffnung (41) eingeführt ist.

10. Einführinstrument zum Einsetzen einer Befestigungsvorrichtung (1; 100) für eine Sehne oder ein Band nach einem der vorhergehenden Ansprüche, umfassend ein Einsatzende (23, 26) das zum Einsetzen in eine Durchgangsöffnung (9) eines ersten Bereichs (2) der Vorrichtung (1; 100) eingerichtet ist, wobei das Einsatzende eine Spitze (23) mit einem nicht kreisförmigen Querschnitt, der so konfiguriert ist, dass dieser mit einem ersten Abschnitt (9a) der Öffnung (9) im Eingriff steht, und eine geformte Spitze koaxial mit der Spitze (23) und mit einem nicht kreisförmigen Querschnitt umfasst, der dem Querschnitt der Spitze (23) entspricht, die zum drehbaren Aufnehmen in einen zweiten Abschnitt (9b) der Öffnung (9) vorgesehen ist, wobei die geformte Spitze (26) in Bezug auf die Spitze (23) zwischen einer ausgerichteten Position, an der die Spitze (23) und die geformte Spitze (26) ausgerichtet sind, um in die Öffnung (9) eingeführt /aus dieser herausgezogen zu werden, und einer nicht ausgerichteten Position drehbar ist, an der die Spitze (23) und die geformte Spitze (26) nicht ausgerichtet sind, wobei Bereiche der geformten Spitze (26) in Bezug auf den Querschnitt der Spitze (23) vorragen und die geformte Spitze (26) im zweiten Abschnitt (9b) verriegelt ist.

11. Revisionswerkzeug (30) für die Befestigungsvorrichtung (1) für eine Sehne oder ein Band nach einem der Ansprüche 1 bis 9, umfassend einen Körper (32), der stabförmig und innen hohl ist, um eine mit einem Griffende (34) versehene Stange (33) verschiebbar aufzunehmen, die zum Zusammenwirken mit der zweiten Vorrichtung (3) vorgesehen ist, wobei das Greifende (34) ein Paar von Greifarmen (36a, 36b) aufweist, die voneinander weg/zueinander hin bewegbar sind, wobei der stabförmige Körper (32) und der Stab (33) relativ zueinander entlang einer Längsachse (X') des Werkzeugs zwischen einer herausgezogenen Position (Y2), an der das Greifende (34) außerhalb des stabförmigen Körpers (32) angeordnet ist und die Greifarme (36a, 36b) derart voneinander weg bewegt sind, dass zwischen diesen ein Sitz zum Aufnehmen eines Endes (4) des zweiten Vorrichtungsbereichs (3) definiert ist, und einer eingezogenen Position (Y1) bewegbar sind, an der die Stange (33) fast vollständig im Innern des stabförmigen Körpers (32) positioniert ist und die Greifarme (36a, 36b) zueinander hin bewegt wurden, um das Ende (4) des zweiten (3) Vorrichtungsbereichs festzuklemmen.

12. Revisionswerkzeug (30') für die Befestigungsvorrichtung (100) für eine Sehne oder ein Band nach einem der Ansprüche 1 bis 7, umfassend einen Handgriffbereich (31'), der zum Greifen durch einen Bediener zum Handhaben des Werkzeugs vorgesehen ist, wobei ein stabförmiger Körper (32') einen Innenhohlraum (42') zum verschiebbaren Aufnehmen einer mit einem Gewindeende (34') versehenen Stange (33') definiert, die zum Zusammenwirken mit dem zweiten Vorrichtungsbereich (3) vorgesehen ist, wobei der Hohlraum (42') am gegenüberliegenden Ende in Bezug auf den Handgriffbereich (31') einen Anschlussbereich (421) mit vergrößertem Querschnitt aufweist, der so konfiguriert ist, dass dieser den Schaft (11') des zweiten Vorrichtungsbereichs (3) mit einer Verdrehsicherungsverbindung aufnimmt, wobei das Gewindeende (34') zum Einschrauben in eine Gewindeöffnung (12") des Schafts (11' vorgesehen ist.

## Revendications

1. Dispositif de fixation pour un tendon ou un ligament, comprenant :
- une première partie de dispositif (2) adaptée pour être fixée à un os d'une personne,
- une seconde partie de dispositif (3) dotée d'un appendice d'engagement (4 ; 4') pour maintenir la tension du tendon ou du ligament à fixer, et couplée de manière stable à la première partie de dispositif (2) afin d'éviter que le tendon ou le ligament, ne se désengage de l'os,
- la seconde partie (3) étant couplée à la première partie (2) de manière librement rotative afin de permettre la libre rotation relative entre la première (2) et la seconde (3) partie autour d'un axe longitudinal (X) du dispositif (1),
**caractérisé en ce que** la seconde partie (3) est translatable de manière limitée par rapport à la première partie (2) entre une première position opérationnelle (Z), dans laquelle la seconde partie (3) est librement rotative par rapport à la première partie (2) et une seconde position opérationnelle (W), dans laquelle la seconde partie (3) est fixée pour la rotation avec la première partie (2) et **en ce qu'**il comprend des moyens de fixation, comprenant une surface externe d'une tige (11, 11') de la seconde partie (3), et des moyens de contre-fixation correspondants, comprenant une cavité de logement (9e) pour la tige (11 ; 11') définie dans la première partie (2), la tige (11 ; 11') étant au moins en partie insérée, dans la seconde position (W), en engagement antirotation dans la cavité de logement (9e) afin de fixer la première (2) et la seconde (3) partie l'une à l'autre pour la rotation autour de l'axe longitudinal (X).

2. Dispositif de fixation selon la revendication 1, dans lequel la seconde partie (3) est translatable de manière limitée le long de l'axe longitudinal (X) afin d'être déplacée entre la première position opérationnelle (Z) et la seconde position opérationnelle (W).

3. Dispositif de fixation selon la revendication 1, dans lequel la première partie (2) comprend une capsule (7) s'étendant le long de l'axe longitudinal (X) et dotée d'un filet externe (8) pour visser la capsule (7) à un os d'un patient, et avec un trou débouchant (9) présentant une section transversale variable le long de l'axe longitudinal (X), la cavité de logement étant définie dans une section (9e) du trou débouchant (9).

4. Dispositif de fixation selon la revendication précédente, dans lequel la tige (11 ; 11') s'étend depuis l'appendice d'engagement (4 ; 4') et est dotée d'un trou fileté (12), la seconde partie de dispositif (3) comprenant aussi une vis (13) adaptée pour être insérée de manière librement rotative dans le trou débouchant (9) et dotée d'une tige (15) adaptée pour être au moins en partie vissée dans le trou fileté (12) de manière à coupler la seconde partie (3) et la première partie (2) l'une à l'autre.

5. Dispositif de fixation selon l'une quelconque des revendications 3 à 4, dans lequel la tige (11 ; 11') et la section (9e) du trou débouchant (9) ont des sections transversales non circulaires similaires de sorte que lorsque la tige (11 ; 11') est logée dans la section (9e), la première (2) et la seconde (3) partie de dispositif soient fixées l'une à l'autre pour la rotation autour de l'axe longitudinal (X).

6. Dispositif de fixation selon l'une quelconque des revendications 3 à 5, dans lequel le trou débouchant (9) comprend une première section (9a) qui est prévue sur l'extrémité en regard par rapport à la seconde partie (3) et qui est destinée à recevoir un bout (23) d'un instrument d'insertion (20), la première section (9a) et le bout (23) étant configurés de manière à s'engager l'un avec l'autre dans un couplage d'antirotation.

7. Dispositif de fixation selon la revendication précédente, dans lequel le trou débouchant (9) comprend aussi une seconde section (9b) adjacente à la première section (9a) et présentant une section transversale circulaire entourant au moins la section transversale de la première section (9a) de manière à recevoir un bout formé (26) de l'instrument d'insertion (20) et à verrouiller le bout formé (26) en translation par rapport au dispositif (1).

8. Dispositif de fixation selon l'une quelconque des revendications précédentes, dans lequel l'appendice d'engagement présente la forme d'un oeillet (4, 4') fixé à la tige (11 ; 11').

9. Dispositif de fixation selon l'une quelconque des revendications 1 à 7, dans lequel l'appendice d'engagement présente la forme d'un anneau (4') inséré dans un trou (41) prévu dans la tige (11').

10. Instrument d'insertion pour insérer un dispositif de fixation (1 ; 100) pour un tendon ou un ligament selon l'une quelconque des revendications précédentes, comprenant une extrémité d'insertion (23, 26) agencée pour être insérée dans un trou débouchant (9) d'une première partie (2) du dispositif (1 ; 100), l'extrémité d'insertion comprenant un bout (23) présentant une section transversale non circulaire configurée de manière à engager une première section (9a) du trou (9), et un bout formé (26) coaxial avec le bout (23) et présentant une section transversale non circulaire correspondant à la section transversale du bout (23), qui est destiné à être logé de manière rotative dans une seconde section (9b) du trou (9), le bout formé (26) étant rotatif par rapport au bout (23) entre une position d'alignement, dans laquelle le bout (23) et le bout formé (26) sont alignés afin d'être insérés/extraits du trou (9) et une position de non alignement, dans laquelle le bout (23) et le bout formé (26) ne sont pas alignés, des parties du bout formé (26) faisant saillie par rapport à la section transversale du bout (23) et le bout formé (26) étant verrouillé dans la seconde section (9b).

11. Outil de révision (30) pour le dispositif de fixation (1) pour un tendon ou ligament selon l'une quelconque des revendications 1 à 9, comprenant un corps (32) qui est en forme de tige et creux à l'intérieur afin de loger de manière coulissante une tige (33) dotée d'une extrémité de saisie (34) qui est destinée à interagir avec la seconde partie de dispositif (3), l'extrémité de saisie (34) comprenant une paire de bras de saisie (36a, 36b) capables d'être déplacés loin l'un de l'autre et l'un vers l'autre, le corps en forme de tige (32) et la tige (33) étant mobiles l'un par rapport à l'autre le long d'un axe longitudinal (X') de l'outil entre une position extraite (Y2), dans laquelle l'extrémité de saisie (34) est située en dehors du corps en forme de tige (32) et les bras de saisie (36a, 36b) sont déplacés loin l'un de l'autre de manière à ce qu'il soit défini entre eux un siège pour loger une extrémité (4) de la seconde partie de dispositif (3), et une position rétractée (Y1), dans laquelle la tige (33) est presque complètement positionnée dans le corps en forme de tige (32) et les bras de saisie (36a, 36b) ont été déplacés l'un vers l'autre afin de serrer l'extrémité (4) de la seconde partie de dispositif (3).

12. Outil de révision (30') pour le dispositif de fixation (100) pour un tendon ou un ligament selon l'une quelconque des revendications 1 à 7, ou 9, comprenant une partie de manche (31') qui est destinée à être saisie par l'utilisateur afin de manipuler l'outil, un corps en forme de tige (32') définissant une cavité interne (42') pour recevoir de manière coulissante une tige (33') dotée d'une extrémité filetée (34') qui est destinée à interagir avec la seconde partie de dispositif (3), la cavité (42') comprenant, sur l'extrémité opposée par rapport à la partie de manche (31'), une partie terminale (421) de section transversale agrandie configurée de manière à recevoir avec un couplage d'antirotation la tige (11') de la seconde partie de dispositif (3), l'extrémité filetée (34') étant destinée à être vissée dans un trou fileté (12") de la tige (11').
